# EUROPEAN PATENT SPECIFICATION

(11) **EP 2 323 682 B1**
(45) Date of publication and mention of the grant of the patent: **30.04.2014**
(21) Application number: 09800806.3
(22) Date of filing: 14.07.2009
(51) Int. Cl.: A61K 38/38, A61K 38/40, A61P 29/00, A23L 1/30, A61K 33/30, A61P 39/06, A61K 38/57, A61K 38/17

(54) **COMPOSITIONS COMPRISNG ZINC-CHARGED PROTEIN FOR INCREASING SERUM ANTIOXIDANT CONCENTRATIONS**
ZUSAMMENSETZUNGEN ENTHALTEND ZINC-BELADENE PROTEINE ZUR ERHÖHUNG DER ANTIOXIDANTIENKONZENTRATION IN SERUM
COMPOSITIONS CONTENANT UNE PROTÉINE CHARGÉE EN ZINC POUR AUGMENTER LES CONCENTRATIONS D'ANTIOXYDANT DANS LE SÉRUM

(30) Priority: 25.07.2008 US 180504
(43) Date of publication of application: 25.05.2011
(73) Proprietor: Ambryx Biotechnology, Inc, Riverside, CA 92507 (US)
(72) Inventor: TSAI, Men, Hwei, Diamond Bar CA 91765 (US)
(74) Representative: Kling, Simone
(86) International application number: PCT/US2009/050547
(87) International publication number: WO 2010/011533

(56) References cited:
- US-A1- 2003 165 574
- US-A1- 2005 282 738
- US-A1- 2006 008 544
- US-A1- 2007 004 617

## Description

### BACKGROUND OF THE INVENTION

### Field of the Invention

The invention relates to compositions for increasing serum antioxidant levels, increasing serum ghrelin levels, and decreasing serum TNF-alpha levels. In particular, the compositions comprise a mixture a mixture of zinc-charged, protease digested proteins derived, for example, from serum or milk.

### Description of the Related Art

Oxidative chemical species are unstable, reactive molecules such as free-radicals and reactive oxygen species that may attack cell structures in the body. Higher than normal serum concentrations of oxidative chemical species have been linked to premature aging and an increased risk of certain kinds of cancers.

Antioxidants are a class of chemicals which, under certain circumstances, may act to fully or partially neutralize oxidative chemical species-in some case reducing or eliminate the health hazards presented by oxidative molecules. Thus, the presence of above normal serum concentrations of antioxidants, or the consumption of antioxidants, can reduce oxidative stress levels and may be associated with longevity in humans and other mammals and animals. It is therefore desirable to develop simple methods for increasing antioxidant levels to combat the deleterious effects of free-radicals, reactive oxygen species, and other chemical species capable of causing oxidative stress on mammalian biochemistry and physiology.

Low fasting insulin levels are also associated with longevity in human and mammals, such as mice (Science 299:572, 2003; Science 297: 811 2002). Mutations that inhibit the insulin and IGF-1 signaling pathways increase life span in worms, flies, and mammals including human (Science 299, 1346-1351, 2003; Proc Nat Acad Sci 105, 3438-3442, 2008). Furthermore, blocking of the insulin receptor signaling pathway increases expression of antioxidant genes, leading to the increased production of superoxide dismutase, for example, which acts to catalytically remove superoxide. Thus, various studies seem to indicate that inhibition of insulin receptor activity leads to the activation of antioxidant genes, which in turn lowers oxidative stress and extends lifespan (Cell 120, 449-460. 2005). In addition, mice with disrupted insulin receptor signaling also showed decreased age-related glucose intolerance and triglycerides levels, further contributing to longevity (Science 299:572-574, 2003).

Severe weight loss is a symptom of many diseases and, oftentimes, it can exacerbate an already precarious medical condition. For example, more than 60% of advanced cancer patients suffer from anorexia and cachexia (Curr Opin Clin Nutr Metab Care. 2007 Jul;10(4):443-8), and, so far, there is a large unmet need in helping cancer patients maintain weight.

Ghrelin is a hormone released by the stomach. It is a 28-amino acid peptide which has been shown to up-regulate body weight through appetite stimulation, down-regulation of energy expenditure, and induction of adiposity. Furthermore, ghrelin inhibits pro-inflammatory cytokines such as IL-1alpha, IL-1beta, TNF-alpha which may cause oral mucositis and anorexia, which are the results of weight loss. Recently, ghrelin has been shown to be beneficial for cancer patients. See Barclay, J. Clin. Endocrinol. Metab. 89:2832 - 2836 (2004). Ghrelin mimetics are also being developed to combat cachexia (Oncologist: 2007 May;12(5):594-600).

### SUMMARY OF THE INVENTION

The compositions are as defined in the appended claims.

### BRIEF DESCRIPTION OF THE DRAWINGS

Figure 1 illustrates the effect of a disclosed composition derived from porcine serum on mouse insulin receptor signaling activity.
Figure 2 illustrates the therapeutic effect of a disclosed composition on human serum free radicals levels one day after ingestion of the composition.
Figure 3 illustrates the therapeutic effect of a disclosed composition on human serum antioxidant levels four days and six days after ingestion of the composition.
Figure 4 illustrates the therapeutic effect of a disclosed composition on the activity of superoxide dismutase in human serum at 25 fold dilution six days after ingestion of the composition.
Figure 5 illustrates the therapeutic effect of a disclosed composition on the activity of superoxide dismutase in human serum at 500 fold dilution six days after ingestion of the composition.
Figure 6 illustrates the therapeutic effect of a disclosed composition on human serum fasting insulin levels over a period of approximately one month during which the composition was ingested.
Figure 7 illustrates the therapeutic effect of a disclosed composition on human serum antioxidant levels over a period of approximately one month during which the composition was ingested.
Figure 8 illustrates the therapeutic effect of a disclosed composition on human urine free radical levels over a period of approximately one month during which the composition was ingested.
Figure 9 illustrates the therapeutic effect of a disclosed composition on the ability of human serum to inhibit free radical formation at the end of a three year period during which the composition wasw ingested.
Figure 10 illustrates the therapeutic effect of a disclosed composition on the life expectancy of *C*. *elegans.*
Figure 11 illustrates the therapeutic effect of a disclosed composition on human serum ghrelin levels four days and six days after ingestion of the composition.
Figure 12 illustrates the therapeutic effect of a disclosed composition on human serum TNF-alpha levels four days and six days after ingestion of the composition.
Figure 13 illustrates the therapeutic effect of a disclosed composition on human serum triglycerides levels eight days after daily ingestion of the composition, and 2 days and 10 days triglycerides levels after stopping further ingestion of the composition.

### DETAILED DESCRIPTION

The application describes compositions for increasing serum antioxidant levels, increasing serum ghrelin levels, and decreasing serum TNF-alpha levels. In particular, the application discloses preparing and administering to a patient a mixture of zinc-charged, protease digested proteins derived, for example, from serum or milk.

While not intending to be limited to any particular theory of biological activity, various compositions described herein are shown to inhibit cellular insulin receptor signaling activity, which in turn upregulates expression of antioxidant genes, chaperones, and antimicrobial genes which can protect cells from environmental stress. For example, blocking of insulin receptor signaling pathway increases expression of antioxidant genes such as superoxide dismutase and decrease serum triglycerides levels. In some embodiments, the mechanism of action involves inhibition of insulin receptor on fat cells, which can activate superoxide dismutase (SOD) through a still unknown mechanism which leads to increase in serum antioxidant level.

Fetal and adult animal (cow, sheep, mouse, and rat) serum as well as fetal and adult human serum contain zinc-binding proteins, including fetuin, α-1-acid glycoprotein (AAG), α-1-antitrypsin (AAT). A composition comprising one or more of these proteins may be supercharged with zinc so that, after supercharging, the proteins contain a higher quantity of bound zinc than they would have bound in their natural state. It has been discovered that a composition comprising such supercharged zinc binding proteins has the ability to increase a subject's serum antioxidant levels, upon administration of a sufficient dose. In addition, a mixture of peptide fragments resulting from the fragmentation, such as through protease digestion, of one or more zinc-charged zinc-binding proteins may retain the capacity to increase a subject's serum antioxidant levels. If not charged with zinc, both the zinc-binding proteins and the digested peptide fragments have a greatly reduced capacity to increase antioxidant levels. While not intending to be bound by any particular theory of operation, it is believed that zinc-charging is, in part, a basis for the therapeutic activity of the compositions of the methods. The zinc-charging, as described in the sections below, yields compositions with substantially different bound zinc than, for example, native serum or milk proteins.

It is now further disclosed that, in addition to serum, antioxidant level promoting zinc supercharged proteins and protein fragments can be derived from milk. When whole milk was treated with zinc and protylytic enzymes yielding zinc-charged protein fragments, the fragments were found to increase a subject's serum antioxidant levels upon administration of a sufficient dose of a composition comprising the fragments.

Also disclosed herein are the results of various experiments and studies illustrating the therapeutic effects of some of the example compositions disclosed herein. These experiments and studies have shown, among other things, the short term (e.g. twenty-four hour, four day, six day) therapeutic effects of ingestion of some of the disclosed compositions, the mid-term (e.g. one month) therapeutic effects of ingestion of some of the disclosed compositions, and the long-term (e.g. three year) therapeutic effects of ingestion of some of the disclosed compositions. The therapeutic effects caused by ingestion, as illustrated in these studies, include increases in serum antioxidant concentrations, inhibition of free-radical formation, decreases in urine concentration of oxidative chemical species, increases in the activity levels of superoxide dismutase in serum, decreases in fasting serum insulin concentrations, decrease in triglycerides concentrations, increases in serum ghrelin concentrations, and decreases in serum TNF-alpha concentrations. In particular, serum free radical formation was shown to decrease twenty-four hours after ingestion of a disclosed composition. Also shown was that ingestion of a disclosed composition over an approximately one month period can increase serum antioxidant levels by approximately 63% and decrease urine free radical levels by approximately 46% as measured by various times throughout the month.

### Definitions

This patent application often refers to compositions with the capability (upon administration to a subject) of increasing serum antioxidant levels. However, increasing serum antioxidant levels is generally accompanied by a reduction in the serum levels of oxidative chemical species. Therefore, the phrase "increasing serum antioxidant levels" or "promoting antioxidant levels" and similar phrases should be construed as equivalently referring to decreases in levels of oxidative species, or reductions in serum concentration of oxidative species, or reduced oxidative stress, or similar phrases. The converse is also true: references to decreases in oxidative chemical species or oxidative stress also refer to increases in serum antioxidant levels.

As used herein, and unless otherwise indicated, the term "protein" refers to a molecule comprising one or more polypeptide chains, or a fragment thereof. Since the polypeptide chain can be modified with, for example, one or more saccharides or phosphates, the term protein specifically includes glycoproteins and post-translational modification (PTM) containing proteins. The term protein, unless indicated otherwise, has no size limitation. In particular, the term includes protease digested fragments of proteins, including glycogroteins, from, for example, serum or milk. Preferred proteases for digestion include papain.

As used herein and unless otherwise indicated, the terms "treat," "treating" and "treatment" refer to alleviating or reducing the severity of a symptom associated with the disease or condition being treated.

As used herein and unless otherwise indicated, the terms "manage," "managing" and "management" refer to maintaining a reduction in severity or avoidance of a symptom associated with the disease or condition being managed.

As used herein and unless otherwise indicated, the terms "prevent," "preventing" or "prevention" refers to reducing risk of or delaying the onset of acquiring a disease or condition being prevented, or a symptom thereof.

As used herein "subject" is an animal, typically a mammal. In preferred embodiments, the subject is a human, such as a patient.

As used herein, and unless otherwise specified, the terms "therapeutically effective amount" and "effective amount" of a compound refer to an amount sufficient to provide a therapeutic benefit in the treatment, prevention and/or management of a disease, to delay or minimize one or more symptoms associated with the disease or disorder to be treated. The terms "therapeutically effective amount" and "effective amount" can encompass an amount that improves overall therapy, reduces or avoids symptoms or causes of disease or disorder, or enhances the therapeutic efficacy of another therapeutic agent

The terms "co-administration" and "in combination with" include the administration of two therapeutic agents either simultaneously, concurrently or sequentially with no specific time limits. In one embodiment, both agents are present in a subject at the same time or exert their biological or therapeutic effect at the same time. In one embodiment, the two therapeutic agents are in the same composition or unit dosage form. In another embodiment, the two therapeutic agents are in separate compositions or unit dosage forms.

The term "dietary supplement" refers to materials defined as dietary supplements in Section 3 of the Dietary Supplement Health and Education Act of 1994, Public Law 103-417, Oct. 25, 1994. A dietary supplement is a product taken by mouth that contains a "dietary ingredient" intended to supplement the diet. The "dietary ingredients" include the zinc-charged, protease digested compositions of the invention and optionally one or more other dietary ingredients such as vitamins, minerals, herbs or other botanicals, amino acids, and substances such as enzymes, organ tissues, glandulars, and metabolites. Dietary supplements can also be extracts or concentrates, and may be found in many forms such as tablets, capsules, softgels, gelcaps, liquids, or powders.

### Compositions and Preparation Thereof

Provided herein are compositions that can be used to increase serum antioxidant levels in a subject

Disclosed herein are compositions comprising a zinc-charged, protease digested serum or milk protein in an effective amount for use in increasing serum antioxidant levels.

The protein can be from any source apparent to those of skill in the art. In certain embodiments, the protein is isolated from blood serum. In certain embodiments, the protein is isolated from milk. In certain embodiments, the serum proteins are not isolated from serum, and are zinc-charged and protease digested while remaining in serum In certain embodiments, the milk proteins are not isolated from milk, and are zinc-charged and protease digested while remaining in milk. The serum or milk can be but not limited to from an animal such as a cow, sheep, pig, horse or human. The blood serum can be adult or fetal blood serum. The milk or serum can be obtained and/or prepared according to any technique apparent to those of skill in the art.

In certain embodiments, the protein can be produced and isolated from recombinant sources according to techniques known to those of skill in the art. In certain embodiments, the protein can be produced by synthetic or semi-synthetic techniques known to those of skill in the art. In further embodiments, the protein can be obtained from a commercial source.

The milk or serum protein can be any milk or serum protein effective for the methods provided herein. In certain embodiments, the serum or milk protein is selected from the group consisting of α-2-HS-glycoprotein, α-1 -acid glycoprotein, α-1-antitrypsin, albumin and transferrin. In certain embodiments, the serum or milk protein is α-2-HS-glycoprotein. In certain embodiments, the serum or milk protein is α-1-acid glycoprotein. In certain embodiments, the serum or milk protein is α-1-antitrypsin. In certain embodiments, the serum or milk protein is albumin. In certain embodiments, the serum or milk protein is transferrin.

In certain embodiments, the composition comprises more than one serum or milk protein. The proteins may be selected from the group consisting of α-2-HS-glycoprotein, α- 1 -acid glycoprotein, a-1-antitrypsin, albumin, transferrin and a-fetoprotein. In certain embodiments, the composition comprises two serum or milk proteins selected from the group consisting of α-2-HS-glycoprotein, α-1-acid glycoprotein, a-1-antitrypsin, albumin, transferrin and α-fetoprotein. In certain embodiments, the composition comprises three serum or milk proteins selected from the group consisting of α-2-HS-glycoprotein, α-1-acid glycoprotein, α-1-antitrypsin, albumin, transferrin and a-fetoprotein. In certain embodiments, the composition comprises four serum or milk proteins selected from the group consisting of α-2-HS-glycoprotein, α-1-acid glycoprotein, α-1-antitrypsin, albumin, transferrin and α-fetoprotein. In certain embodiments, the composition comprises five serum or milk proteins selected from the group consisting of α-2-HS-glycoprotein, α-1 -acid glycoprotein, α-1-antitrypsin, albumin, transferrin and α-fetoprotein.

In some formulations, the serum or milk protein may be zinc-charged. As discussed below, zinc-charging may yield a composition with bound zinc that is substantially different from the trace amount of zinc that might be present in native serum or milk. The serum or milk protein may be zinc-charged according to any technique apparent to those of skill in the art. Exemplary techniques are described, for example, in U.S. Patent Nos. 5,994,298, 6,258,779, 6,720,311 and 6,737,402. In some embodiments, proteins may be zinc-charged by removing bound ions, such as metal ions, followed by contacting the proteins with zinc ion. Bound ions can be removed by any technique apparent to those of skill in the art including, for example, dialysis, filtration, chelating agents, etc. In certain embodiments, bound ions are removed by contacting the proteins with an effective amount of a chelating agent such as EDTA, EGTA, trisodium citrate, or nitriolotriacetic (NTA)-based agents. Conditions may include, for example, about 1 milimolar ("mM") to about 100 mM EDTA for about 40 micromolar ("µM") protein. In certain embodiments, following removal of bound ions the chelating agent is removed from the proteins by a standard technique such as filtration, dialysis, chromatography or molecular sieves.

The proteins may be charged with zinc by contact with zinc in any form and in any amount which is effective to charge the proteins with zinc. Exemplary forms of zinc include zinc salts such as zinc chloride, zinc acetate and zinc sulfate. In the working examples below, the proteins are charged with zinc acetate. Conditions may include, for example, about 1mM to about 500 mM zinc salt, e.g. zinc acetate, zinc chloride or zinc sulfate, for 40 µM protein. Conditions may include about 1 to about 500 mM zinc acetate for 40 µM protein. In certain embodiments, excess zinc may be removed from the proteins by standard techniques such as filtration, dialysis, chromatography or molecular sieves.

In the compositions disclosed herein, the proteins may be protease digested. The proteins can be protease digested prior to zinc-charging, during zinc-charging or following zinc charging. In the working examples below, zinc-charging is followed by protease digestion. The protease can be any protease known to those of skill in the art that is capable of generating fragments which may be effective at increasing antioxidant levels. Exemplary proteases include papain. The proteins are contacted with the protease under conditions suitable for generating fragments effective in the methods of the invention. In certain embodiments, the proteins are contacted with papain at 37°C for about two hours or more. In certain embodiments, papain is removed from the proteins by standard techniques such as filtration, dialysis, chromatography or molecular sieves. In certain embodiments, the proteins can be digested by other techniques known to those of skill in the art, such as a freeze-thaw technique. Digestion of the proteins results in fragmenting of the proteins and creates protein fragments. In general, fragmentation of proteins in connection with the various embodiments of the invention can be accomplished in many ways. These include digestion by protease, fragmentation via freeze-thaw methods, and other methods that will be known to those having ordinary skill in the art.

In certain embodiments, the zinc-charged, protease digested serum or milk protein as described herein is substantially of a size less than 100 kDa, 50 kDa, 25 kDa, 20 kDa, 15 kDa, 10 kDa, 5 kDa or 3 kDa. The term "substantially of a size less than" indicates no more than 50%, 75%, 90% or 95% of the protease digested serum or milk protein fragments are greater than the size limit. The zinc-charged, protease digested serum or milk protein fragments can be sized according to any technique apparent to those of skill in the art including, for example, filtration, dialysis, chromatography or molecular sieves.

The compositions can be formulated into a form described in the sections below, such as a dietary supplement, a food additive, a food composition, a nutraceutical composition or a pharmaceutical composition, by standard techniques, including those described below.

### Increasing Serum Antioxidant Concentrations, and/or Increasing Serum Ghrelin Concentrations in Mammals: Mammals;

Disclosed herein are methods of increasing serum antioxidant concentrations in a mammalian subject comprising administering to the subject a therapeutically effective amount of one or more compositions. The compositions may alternatively or additionally be administered to a mammalian subject for the purpose of increasing serum ghrelin concentrations. The compositions may alternatively or additionally be administered to a mammalian subject for the purpose of decreasing serum TNF-alpha concentrations.

The composition may comprise a first plurality of zinc-charged protein fragments. In certain such methods, the composition may additionally comprise a second plurality of zinc-charged protein fragments. In certain such methods, the composition may additionally comprise a third plurality of zinc-charged protein fragments. In some methods, the zinc-binding proteins may be proteins native to mammalian milk and/or mammalian serum. In some methods, the zinc-binding proteins may be proteins native to the serum of non-mammalian animals. In some methods, the zinc-binding proteins may be selected from a group consisting of α-2-HS-glycoprotein, α-1-acid glycoprotein, α-1-antitrypsin, albumin, transferrin and α-fetoprotein.

The zinc-charged protein fragments may be created by a process comprising digesting one or more pluralities of zinc-binding proteins with protease. In some methods, the zinc-charged protein fragments (and/or the composition to be administered) may be created by contacting mammalian serum or milk with protease. In some methods, the protease is papain. As discussed above, the zinc-charged protein fragments can also be created by other processes for fragmentation of one or more pluralities of zinc-binding proteins, such as a freeze-thaw technique. The zinc-charged protein fragments may be created by a process comprising contacting one or more pluralities of zinc-binding proteins or fragments thereof with zinc ion. In some methods, the steps of fragmenting and contacting with zinc may be reversed. In some methods, the zinc-charged protein fragments (and/or the composition to be administered) may be created by contacting mammalian serum or milk with zinc ion. The zinc-charged protein fragments may be created by a process comprising both of the steps just described in any order. In some methods, contacting zinc-binding protein with zinc comprises utilizing zinc in the form of a solution of zinc acetate. In other methods, a solution of a different zinc salt is utilized, such as zinc chloride and zinc sulfate. In some methods, the solution of zinc salt(s) (such as zinc acetate, zinc chloride, or zinc sulfate, or any combination thereof) comprises the zinc salt(s) in a concentration of between about 1 mM to about 500 mM (including about 5 mM, 10 mM, 25 mM, 50 mM, 100 mM, 200 mM, 250 mM, 300 mM, 350 mM, 400 mM, and about 450 mM, also including concentrations between these recited concentrations, as well as ranges of concentrations bordered on the low side and the high side by the recited concentrations), In some methods, the process additionally comprises removing excess zinc not bound to any protein.

The process of creating zinc-charged protein fragments may also comprise contacting the one or more pluralities of zinc-binding proteins or fragments with a chelating agent. In some methods, the zinc-charged protein fragments (and/or the composition to be administered) may be created by contacting mammalian serum or milk with a chelating agent. In some methods, the chelating agent comprises EDTA and/or EGTA in a concentration of between about 1mM and about 100mM (including about 5 mM, 10 mM, 15 mM, 20 mM, 25 mM, 30 mM, 35 mM, 40 mM, 45 mM, 50 mM, 60 mM, 70 mM, 80 mM, and about 90 mM, also including concentrations between these recited concentrations, as well as ranges of concentrations bordered on the low side and the high side by the recited concentrations). In some methods, the process additionally comprises removing the chelating agent, the removal optionally comprising filtration and/or dialysis. The process of creating zinc-charged protein fragments may also comprise contacting the one or more pluralities of zinc-binding proteins or fragments or mammalian serum or milk with a carrier, excipient or dilutent.

In some methods, some number of, or all, or substantially all of resulting zinc-binding or zinc-charged protein fragments comprising the composition to be administered may have a molecular weight between about 0.3 kilodaltons and about 50 kilodaltons (including about 0.4 kilodaltons, 0.5 kilodaltons, 0.6 kilodaltons, 0.7 kilodaltons, 0.8 kilodaltons, 0.9 kilodaltons, 1 kilodaltons, 2 kilodaltons, 3 kilodaltons, 4 kilodaltons, 5 kilodaltons, 10 kilodaltons, 15 kilodaltons, 20 kilodaltons, 30 kilodaltons, and about 40 kilodaltons, also including molecular weights between these recited molecular weights, as well as ranges of molecular weights bordered on the low side and the high side by the recited molecular weights).

In some methods, the average molecular weight of the resulting zinc-binding or zinc-charged protein fragments comprising the composition to be administered may have a molecular weight between about 0.3 kilodaltons and about 50 kilodaltons (including about 0.4 kilodaltons, 0.5 kilodaltons, 0.6 kilodaltons, 0.7 kilodaltons, 0.8 kilodaltons, 0.9 kilodaltons, 1 kilodaltons, 2 kilodaltons, 3 kilodaltons, 4 kilodaltons, 5 kilodaltons, 10 kilodaltons, 15 kilodaltons, 20 kilodaltons, 30 kilodaltons, and about 40 kilodaltons, also including molecular weights between these recited molecular weights, as well as ranges of molecular weights bordered on the low side and the high side by the recited molecular weights).

In some methods, the composition to be administered may be substantially free of zinc-charged or zinc-binding protein fragments having a molecular weight greater than a cutoff between about 0.8 kilodaltons and about 50 kilodaltons (including about 0.9 kilodaltons, 1 kilodaltons, 2 kilodaltons, 3 kilodaltons, 4 kilodaltons, 5 kilodaltons, 10 kilodaltons, 15 kilodaltons, 20 kilodaltons, 30 kilodaltons, and about 40 kilodaltons, also including molecular weight cutoffs between these recited molecular weight cutoffs). In some methods, the process of creating the composition may comprise selecting a cutoff and isolating the zinc-charged or zinc-binding protein fragments from some of the molecules having a molecular weight greater than that the selected cutoff. The cutoff may be between about 0.3 kilodaltons and about 50 kilodaltons (including about 0.4 kilodaltons, 0.5 kilodaltons, 0.6 kilodaltons, 0.7 kilodaltons, 0.8 kilodaltons, 0.9 kilodaltons, 1 kilodaltons, 2 kilodaltons, 3 kilodaltons, 4 kilodaltons, 5 kilodaltons, 10 kilodaltons, 15 kilodaltons, 20 kilodaltons, 30 kilodaltons, and about 40 kilodaltons, also including molecular weight cutoffs between these recited molecular weight cutoffs).

In some methods, the composition to be administered may comprise a zinc-binding protein in saline solution at a concentration in the range of about 0.01 µM to about 400 µM (including about 0.05 µM, 0.1 µM, 0.25 µM, 0.5 µM, 1.0 µM, 2.5 µM, 5.0 µM, 10.0 µM, 20.0 µM, 30.0 µM, 40.0 µM, 50.0 µM, 60.0 µM, 70.0 µM, 80.0 µM, 90.0 µM, 100.0 µM, 150.0 µM, 200.0 µM, 250.0 µM, 300.0 µM and about 350.0 µM, also including concentrations between these recited concentrations, as well as ranges of concentrations bordered on the low side and the high side by the recited concentrations).

In some methods, the composition may be a dietary supplement. In some methods the composition may be a food additive or food composition. In some methods, the composition may be a pharmaceutical composition. In some methods, the composition may comprise a pharmaceutically acceptable carrier, excipient, or dilutent. In some methods, the composition may be a nutraceutical composition.

The amount (dosage) of a particular composition that must be administered to a subject to induce an increase in serum antioxidant concentrations or increase serum ghrelin levels, or decrease serum TNF-alpha levels can vary greatly depending on the subject and the particular composition being administered. In some methods, a therapeutically effective amount may be at least about 0.02 milligrams ("mg") of zinc-charged protein fragments per kilogram of body mass of the subject. Thus, when administered to a human, typically having a body mass of approximately 70 kilograms, the composition may be formulated into a unit dosage of at least about 1.4 mg of zinc-charged protein fragments. In some methods, a therapeutically effective amount may be up to about 50 mg of zinc-charged protein fragments per kilogram of body mass of the subject. Thus, when administered to a human, typically having a body mass of approximately 70 kilograms, the composition may be formulated into a daily dosage form of up to about 3.5 g of zinc-charged protein fragments. In certain such methods, a therapeutically effective amount of the composition may be between about 0.02 milligrams and about 50 milligrams of zinc-charged protein fragments per kilogram of body mass of the subject (including about 0.03 mg, 0.04 mg, 0.05 mg, 0.1 mg, 0.25 mg, 0.5 mg, 1.0 mg, 1.5 mg, 2.0 mg, 2.5 mg, 3.0 mg, 4.0 mg, 5.0 mg, 6.0 mg, 7.0 mg, 8.0 mg, 9.0 mg, 10.0 mg, 11.0 mg, 12.0 mg, 13.0 mg, 14.0 mg, 15.0 mg, 20.0 mg, 25.0 mg, 30.0 mg, 35.0 mg, 40.0 mg, and about 45.0 mg per kilogram of body mass of the subject, also including weights per kilogram of body mass of the subject between these recited weights, as well as ranges of weights bordered on the low side and the high side by the recited weights). In certain methods, a therapeutically effective amount of the composition to be administered may be between about 1 mg of zinc-charged protein fragments per day and about 1000 mg of zinc-charged protein fragments per day (including about 2 mg, 3 mg, 4 mg, 5 mg, 10 mg, 25 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, and about 950 mg, also including weights between these recited weights, as well as ranges of weights bordered on the low side and the high side by the recited weights). Selection of proper dosages for administering the composition to a subject is described in greater detail below.

### Subject Populations

The subject can be any subject in need of the composition for use according to the invention. In certain embodiments, the subject is an animal. In certain embodiments, the subject is a mammal. In certain embodiments, the subject is a human. In certain embodiments, the subject is a patient.

One example group of subjects exemplifying a higher than normal risk of experiencing oxidative stress are cancer patients beginning to undergo chemotherapy. Treatment of cancer using chemotherapeutic agents is often accompanied by side effects caused by the occurrence of oxidative stress. Enhanced lipid peroxidation, reduction of antioxidant vitamins, free radical trapping capacity in plasma, and a marked reduction of tissue glutathione (GSH) levels are frequently detected during chemotherapy. The overwhelming production of reactive oxygen species damages healthy tissues and is therefore considered to be one of the causes for the toxic side effects of chemotherapeutic agents. In particular, tissue and cells with a high proliferation rate, such as the epithelium of the gastrointestinal tract, the bone marrow, and hair follicles are especially affected by the oxidative insult Furthermore, reactive oxygen species generated during cancer chemotherapy may also diminish the efficacy of the treatment by interfering with cellular processes, such as cell cycle progression and drug-induced apoptosis, which are important for chemotherapeutic agents to exert their optimal effect on cancer cells.

### Combinations

The methods disclosed herein for increasing serum antioxidant concentration may be combined with other methods for accomplishing other therapeutic effects. For example, the compositions disclosed herein useful for increasing serum antioxidant concentrations may be combined with other active ingredients having other therapeutic effects, thus creating new compositions useful for lowering serum levels of oxidative species while simultaneously treating a second condition depending on the nature of the other active ingredient. Methods of combining different therapeutic agents are well known to those skilled in the art.

### Dietary Supplements and Pharmaceutical Compositions

The compositions administered in the methods disclosed herein can be administered in the form of a food, a food additive, a dietary supplement, a nutraceutical or a pharmaceutical. The compositions may contain a carrier, a dilutent, or an excipient. Depending on the intended use, the carrier, dilutent, or excipient may be chosen to be suitable for human or veterinary use, food, additive, supplement or pharmaceutical use.

In certain embodiments, compositions used in the methods disclosed herein are provided in a mixture with milk for administration to a subject. In certain embodiments, a composition is provided in the form of a dried powder. The composition may be dried according to any technique known to those of skill in the art including lyophilization, freeze drying, spray drying and evaporative drying. The powder may comprise an antioxidant level promoting composition and optionally any carrier, dilutent, excipient or other additive described herein or known to those of skill in the art. In certain embodiments, an antioxidant level promoting composition is provided in the form of a liquid. The liquid can comprise an antioxidant level promoting composition and optionally any carrier, dilutent, excipient or other additive described herein or known to those of skill in the art. The liquid can be any volume deemed useful by one of skill in the art, for instance, a volume convenient for the dosage and route of administration.

As used herein a "food" or "food composition" is a material consisting essentially of protein, carbohydrate and/or fat, which is used in the body of an organism to sustain growth, repair and vital processes and to furnish energy. Foods may also contain supplementary substances such as minerals, vitamins and condiments. The term food includes a beverage adapted for human or animal consumption. As used herein a "food additive" is as defined by the FDA in 21 CFR 170.3(e)(1) and includes direct and indirect additives.

Compositions provided herein can be formulated using standard formulation techniques into gel caps, teas, tablets, etc. *See, e.g.,* Remington's Pharmaceutical Sciences, 18th ed., Mack Publishing, Easton Pa. (1990). Compositions provided herein may be formulated into a dietary supplement, a nutraceutical formulation, or a pharmaceutical preparation for administration to a subject for the purpose of promoting serum antioxidant levels/concentrations as described herein.

Suitable forms of compositions include food additives, food compositions (including beverage compositions) and dietary supplements. The compositions may be added to various foods so as to be consumed simultaneously. As a food additive, the compositions may be used in the same manner as conventional food additives, and thus, only need to be mixed with other food components.

It will be recognized that dietary supplements may not necessarily use the same formulation ingredients or have the same sterile and other FDA requirements as pharmaceutical compositions. The dietary supplements may be in liquid form, for example, solutions, syrups or suspensions, or may be in the form of a product for reconstitution with water or any other suitable liquid before use. Such liquid preparations may be prepared by conventional means such as a tea, health beverage, dietary shake, liquid concentrate, or liquid soluble tablet, capsule, pill, or powder such that the beverage may be prepared by dissolving the liquid soluble tablet, capsule, pill, or powder within a liquid and consuming the resulting beverage. Alternatively, the dietary supplements may take the form of tablets or capsules prepared by conventional means and optionally including other dietary supplements including 'vitamins, minerals, other herbal supplements, binding agents, fillers, lubricants, disintegrants, or wetting agents, as those discussed above. The tablets may be coated by methods well-known in the art. In a preferred embodiment, the dietary supplement may take the form of a capsule or powder to be dissolved in a liquid for oral consumption.

The magnitude of the dietary dose of an active ingredient in the treatment or prevention of a disease or condition will vary with the severity of the disease or condition to be treated and the route of administration. The dose, and perhaps the dose frequency, will also vary according to age, body weight, response, and the past medical history of the consumer or patient. Suitable dosing regimens can be readily selected by those skilled in the art with due consideration of such factors.

The amount of an antioxidant level promoting composition incorporated into a food product will depend on the kind of food and the desired effect. In general, a single serving comprises an amount of the composition that is about 0.1% to about 100% of the food product (including about 0.2%, 0.3%, 0.4%, 0.5%, 1%, 2%, 5%, 10%, 20%, 30%, 40%, 50%, 60%, 70%, 80%, 90%, and about 95% of the food product, also including percentages between these recited percentages, as well as ranges of percentages bordered on the low side and the high side by the recited percentages). In certain embodiments, a food product comprises an antioxidant level promoting composition in an amount of about 1% to about 10% by weight of the food product.

Examples of food include, but are not limited to, confectionery such as sweets (candies, jellies, jams, etc.), gums, bean pastes, baked confectioneries or molded confectioneries (cookies, biscuits, etc.), steamed confectioneries, cacao or cacao products (chocolates and cocoa), dairy products (yoghurt , milk drinks, etc.), frozen confectioneries (ice cream, ices, etc.), beverages (fruit juice, soft drinks, carbonated beverages), health drinks, health bars, and tea (green tea, black tea, etc.).

Compositions disclosed herein, may be packaged or labeled in various ways and for various purposes. For example, if the composition is to be sold as a commercial product, whether as a food product, a dietary supplement, or a pharmaceutical, a label indicating the presence of a composition of the invention may be appropriate. Optionally, the label may identify the chemical constituents of the composition, may identify beneficial properties of the composition, provide instructions for use of the composition, and/or administration of the composition to a subject.

In some embodiments, a composition for administration is a pharmaceutical composition or a single unit dosage form. Pharmaceutical compositions and single unit dosage forms can comprise a prophylactically or therapeutically effective amount of one or more prophylactic or therapeutic agents, and a typically one or more pharmaceutically acceptable carriers or excipients or diluents. In a specific embodiment and in this context, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a dilutent, adjuvant (e.g., Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water may be an appropriate carrier when the pharmaceutical composition is administered intravenously. Saline solutions and aqueous dextrose and glycerol solutions can also be employed as liquid carriers, particularly for injectable solutions. Examples of suitable Pharmaceutical carriers are described in "Remington's Pharmaceutical Sciences" by E.W. Martin. Nutraceutical compositions can, but need not, comprise one or more active or inactive ingredients that are not necessarily considered pharmaceutically acceptable to current practitioners in the art.

A pharmaceutical or nutraceutical composition useful for increasing antioxidant levels (and/or decreasing serum concentrations of oxidative chemical species) can be administered by any route according to the judgment of those of skill in the art, including but not limited to orally, intravenously, intragastrically, intraduodenally, intrapentoneally or intracerebroventricularly.

Typical pharmaceutical or nutraceutical compositions and dosage forms comprise one or more excipients. Suitable excipients are well-known to those skilled in the art of pharmacy, and non-limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical or nutraceutical composition or dosage form depends on a variety of factors well known in the art including, but not limited to, the way in which the dosage form will be administered to a patient and the specific active ingredients in the dosage form. The composition or single unit dosage form, if desired, can also contain minor amounts of wetting or emulsifying agents, or pH buffering agents.

Lactose-free compositions of the invention can comprise excipients that are well known in the art and are listed, for example, in the U.S. Pharmocopia (USP) SP (XXI)/NF (XVI). In general, lactose-free compositions comprise an active ingredient, a binder/filler, and a lubricant in pharmaceutically compatible and pharmaceutically acceptable amounts. Exemplary lactose-free dosage forms comprise an active ingredient, microcrystalline cellulose, pre-gelatinized starch, and magnesium stearate.

Compositions disclosed herein may include pharmaceutical or nutraceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical or nutraceutical compositions and single unit dosage forms can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Such compositions and dosage forms will contain a prophylactically or therapeutically effective amount of a prophylactic or therapeutic agent preferably in purified form, together with a suitable amount of carrier so as to provide the form for proper administration to the patient. The formulation should suit the mode of administration. In a preferred embodiment, the pharmaceutical compositions or single unit dosage forms are sterile and in suitable form for administration to a subject, preferably an animal subject, more preferably a mammalian subject, and most preferably a human subject.

A pharmaceutical or nutraceutical composition for increasing serum antioxidant levels is formulated to be compatible with its intended route of administration. Examples of routes of administration include, but are not limited to, parenteral, e.g., intravenous, intradermal, subcutaneous, intramuscular, subcutaneous, oral, buccal, sublingual, inhalation, intranasal, transdermal, topical, transmucosal, intra-tumoral, intra-synovial and rectal administration. In a specific embodiment, the composition is formulated in accordance with routine procedures as a pharmaceutical or nutraceutical composition adapted for intravenous, subcutaneous, intramuscular, oral, intranasal or tropical administration to human beings. In an embodiment, a pharmaceutical composition is formulated in accordance with routine procedures for subcutaneous administration to human beings. Typically, compositions for intravenous administration are solutions in sterile isotonic aqueous buffer. Where necessary, the composition may also include a solubilizing agent and a local anesthetic such as lignocamne to ease pain at the site of the injection.

Examples of dosage forms include, but are not limited to: tablets; caplets; capsules, such as soft elastic gelatin capsules; cachets; troches; lozenges; dispersions; suppositories; ointment; cataplasms (poultices); pastes; powders; dressings; creams; plasters; solutions; patches; aerosols (*e.g*., nasal sprays or inhalers); gels; liquid dosage forms suitable for oral or mucosal administration to a patient, including suspensions *(e.g.,* aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g.,* crystalline or amorphous solids) that can be reconstituted to provide liquid dosage forms suitable for parenteral administration to a patient.

The composition, shape, and type of dosage forms of the composition will typically vary depending on their use. For example, a dosage form used in the acute treatment of a disorder may contain larger amounts of one or more of the composition it comprises than a dosage form used in the chronic treatment of the same disease. Also, the therapeutically effective dosage form may vary among different types of diseases or disorders. Similarly, a parenteral dosage form may contain smaller amounts of one or more of the active ingredients it comprises than an oral dosage form used to treat the same disease or disorder. These and other ways in which specific dosage forms encompassed by this invention will vary from one another will be readily apparent to those skilled in the art. *See, e.g.,* Remington's Pharmaceutical Sciences, 18th ed., Mack Publishing, Easton PA (1990).

Generally, the ingredients of compositions comprising the composition are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Methods of administering any of the compositions disclosed herein may use dosage forms of any such composition comprising amounts as described above. The dose may be given as a single once-a-day dose or as divided doses throughout the day.

Pharmaceutical compositions disclosed herein that are suitable for oral administration can be presented as discrete dosage forms, such as, but are not limited to, tablets (*e.g.,* chewable tablets), caplets, capsules, and liquids *(e.g.,* flavored syrups). Such dosage forms contain predetermined amounts of active ingredients, and may be prepared by methods of pharmacy well known to those skilled in the art. See generally, Remington's Pharmaceutical Sciences, 18th ed., Mack Publishing, Easton PA (1990).

In certain embodiments, the oral dosage forms are solid and prepared under anhydrous conditions with anhydrous ingredients, as described in detail in the sections above. However, forms for administration may be other than anhydrous, solid oral dosage forms. As such, further forms are described herein.

Typical oral dosage forms are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g.,* powders, tablets, capsules, and caplets) include, but are not limited to, starches, sugars, microcrystalline cellulose, diluents, granulating agents, lubricants, binders, and disintegrating agents.

Examples of excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pregelatinized starch, hydroxypropyl methyl cellulose, (*e.g.,* Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions is typically present in from about 50 to about 99 weight percent of the pharmaceutical composition or dosage form.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103TM and Starch 1500 LM.

Disintegrants are used with the antioxidant promoting compositions disclosed herein to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that can be used in pharmaceutical formulations of the antioxidant promoting compositions disclosed herein include, but are not limited to, agar-agar, alginic acid, calcium carbonate, microcrystalime cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

Lubricants that can be used in pharmaceutical formulations of the antioxidant promoting compositions disclosed herein include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are often used in an amount of less than about 1 weight percent of the pharmaceutical compositions or dosage forms into which they are incorporated.

### Dosage & Frequency of Administration

The amount of the composition which will be effective in the prevention, treatment, management, or amelioration of a disorder or one or more symptoms thereof will vary with the nature and severity of the disease or condition, and the route by which the active ingredient is administered. The frequency and dosage will also 'vary according to factors specific for each patient depending on the specific therapy (*e.g.,* therapeutic or prophylactic agents) administered, the severity of the disorder, disease, or condition, the route of administration, as well as age, body, weight, response, and the past medical history of the patient. Effective doses may be extrapolated from dose-response curves derived from in vitro or animal model test systems. Possible dosages to be administered to a subject, expressed in milligrams of composition per kilogram of body mass of the subject, have been described above. Also described above, are possible dosages expressed as milligrams of composition administered per day to a subject.

In embodiments where pure protein (e.g. recombinant protein or commercially available protein) is the starting material, the dosage may be at the lower end of the ranges described above. In embodiments where protein is prepared from milk or serum, in dietary supplement or food formulations, the dosages may be at the higher end of the ranges described above. The actual dosage can be determined by a practitioner of skill in the art according to, for example, the subjects age, body weight, BMI or other factors. The compositions may be administered as a single dose or divided doses per day. In some embodiments, the daily dose is administered twice daily in equally divided doses. In other embodiments, the daily dose is administered three times per day. In particular embodiments, the daily dose is administered three times per day in equally divided doses. In particular embodiments, the daily dose is administered four times per day in equally divided doses. In certain embodiments, administration of a composition disclosed herein may be repeated daily. In certain embodiments, the administrations may be separated by at least 1 day, 2 days, or 3 days.

An effective amount of a composition described herein will provide therapeutic benefit without causing substantial toxicity. Toxicity of a composition can be determined by standard pharmaceutical procedures in cell cultures or experimental animals, for example, by determining the LD50 (the dose lethal to 50% of the population) or the LD₁₀₀ (the dose lethal to 100% of the population). The dose ratio between toxic and therapeutic effect is the therapeutic index. Compounds which exhibit high therapeutic indices are preferred. The data obtained from these cell culture assays and animal studies can be used in formulating a dosage range that is not toxic for use in human. The dosage of the compounds described herein lies preferably within a range of circulating concentrations that include the effective dose with little or no toxicity. The dosage may vary within this range depending upon the dosage form employed and the route of administration utilized. The exact formulation, route of administration and dosage can be chosen by the individual physician in view of the patient's condition. (See, *e.g.,* Fingl et al., 1996, In: The Pharmacological Basis of Therapeutics, 9th ed., Chapter 2, p. 29, Elliot M. Ross).

### EXAMPLES

### Example Methods of Zinc Charging Proteins

Method 1: Forty milliliters of serum (from various animals) was incubated with 8 ml of 0.1M EDTA (dissolved in water) for 1 hour. EDTA was removed by dialysis against 4 liters of de-ionized water for 3 hours. The EDTA-treated serum was incubated with zinc acetate (at final Zn concentration of 50 mM) for at least 4 hours. The precipitates were removed by centrifugation. The 40 ml of serum was subjected to three rounds of dialysis against de-ionized water to remove excess zinc: first, against 10 liters for 18 hours and then, against fresh 4 liters for another 4 hours.

Method 2: Forty milliliters of milk (from various animals) was incubated with 8 ml of 0.1M EDTA (dissolved in water) for 1 hour. EDTA was removed by dialysis against 4 liters of de-ionized water for 3 hours. The EDTA-treated milk was incubated with zinc acetate (at final Zn concentration of 50 mM) for at least 4 hours. The precipitates were removed by centrifugation. The 40 ml of milk was subjected to two rounds of dialysis against de-ionized water to remove excess zinc: first, against 10 liters for 18 hours and then, against fresh 4 liters for 4 another hours.

Method 3: A protein solution (40 µM each of a -2-HS-glycoprotein (AHSG, or fetuin), α-1-acid glycoprotein (AAG), α-1-antitrypsin (AAT), albumin, transferrin and α-fetoprotein in any combination or single protein type) was incubated with 10 mM EDTA in a 10 ml saline solution containing 2 mM HEPES, pH 7.0, for 1 hour at room temperature. EDTA was removed by repetitive filtration through a molecular sieve with a molecular weight cut-off of 10 KDa. The protein solution (40 µM) was incubated with 50 mM zinc acetate for 18 hours. The excess zinc ions were removed by four rounds of concentration against 20 volumes of saline solution (pH 7.0), each time using a molecule sieve, which resulted in a dilution factor of 6x10⁻⁶ of zinc ions. The free zinc ion concentration was determined from the 10 KDa membrane filtrate of the protein solution to be about 5-10 µM, a concentration at which no cytotoxicity had been previously observed.

Method 4: A protein solution (40 µM each of α-2-HS-glycoprotein (AHSG, or fetuin), α-1-acid glycoprotein (AAG), α-1-antitrypsin (AAT), albumin, transferrin and α-fetoprotein) was incubated with 5 mM EDTA in a 10 ml saline solution containing 2 mM HEPES, pH 7.0, for 1 hour at room temperature. EDTA was removed by repetitive filtration through a molecular sieve with a molecular weight cut-off of 10 KDa. The protein solution (40 µM) was incubated with 80 µM of zinc acetate for 18 hours.

Method 5: A protein solution (40 µM each of α-2-HS-glycoprotein (AHSG, or fetuin), α-1-acid glycoprotein (AAG), α-1-antitrypsin (AAT), albumin, transferrin and α-fetoprotein) was incubated with 10 mM EDTA in a 10 ml saline solution containing 2 mM HEPES, pH 7.0, for 1 hour at room temperature. EDTA was removed by repetitive filtration through a molecular sieve with a molecular weight cut-off of 10 KDa. The protein solution (40 µM) was incubated with 50 mM zinc acetate for 18 hours. The excess zinc ions were removed by dialysis against 1000 volumes of de-ionized water for 2 hours.

Method 6: A protein solution (40 µM each of α -2-HS-glycoprotein (AHSG, or fetuin), α-1-acid glycoprotein (AAG), α-1-antitrypsin (AAT), alpha-fetoprotein, albumin and transferrin) is incubated with 10 mM EDTA in a 10 ml saline solution containing 2 mM HEPES, pH 7.0, for 1 hour at room temperature. The protein solution (40 µM) is incubated with 50 mM zinc acetate for 18 hours.

### Example Methods of Digestion of Zinc-Charged Proteins With Protease

Method 1: Zinc-charged serum prepared as described above (40 ml) was incubated with papain (2,500 units, from papaya latex) for 2 hours at 37°C. The fragments were collected by passing the incubation mixture through a molecular sieve with a molecular weight cut-off at 3 KDa and up to 10 KDa.

Method 2: Zinc-charged milk prepared as described above (40 ml) was incubated with papain (2,500 units, from papaya latex) for 2 hours at 37°C. The fragments were collected by passing the incubation mixture through a molecular sieve with a molecular weight cut-off at 3 KDa and up to 10 KDa.

Method 3: Zinc-charged proteins prepared as described above (5 ml containing 40 µM each of α-2-HS-glycoprotein (AHSG, or fetuin), α-1-acid glycoprotein (AAG), α-1-antitrypsin (AAT), albumin, transferrin and a-fetoprotein) were incubated with papain (468 units, from papaya latex) for 2 hours at 37°C. The fragments were collected by passing the incubation mixture through a molecular sieve with a molecular weight cut-off at 3 KDa.

Method 4: Zinc-charged proteins prepared as described above (5 ml containing 40 µM each of α-2-HS-glycoprotein (AHSG, or fetuin), α-1-acid glycoprotein (AAG), α-1-antitrypsin (AAT), alpha-fetoprotein, albumin and transferrin) are incubated with papain (468 units, from papaya latex) for 2 hours at 37°C.

### Mammalian Study Showing Inhibition of Insulin Receptor Activity on Mouse Fat Cells

A composition was prepared by the following method: Porcine serum was incubated with EDTA at final EDTA concentration of 10mM for 1 hour. EDTA was removed by dialysis against de-ionized water for 3 hours. The EDTA-treated serum was incubated with zinc acetate (at final Zn concentration of 50 mM) for at least 4 hrs. The precipitates were removed by centrifugation, followed by dialysis against de-ionized water. Zinc-charged serum was then incubated with papain (60 units per milliliter milk mixture, from papaya latex) for 2 hours at 37°C. The fragments were collected by passing the incubation mixture through a molecular sieve with a molecular weight cut-off at 3 KDa.

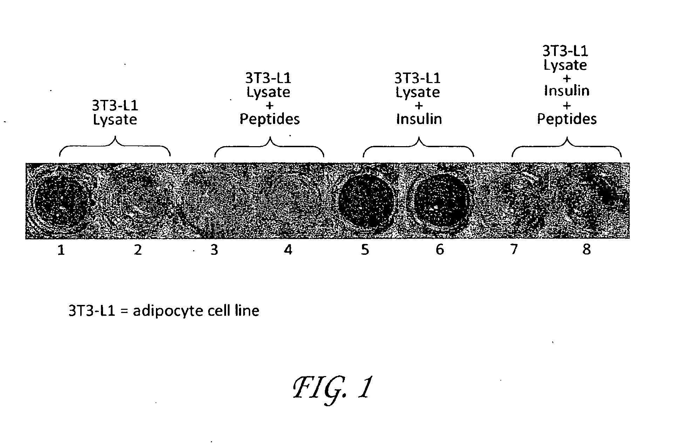

Following preparation of the composition, cell lysate of mouse adipocyte cell line 3T3-L1 which contains abundant insulin receptors was contacted with the composition. The results of this experiment are shown in Figure 1, which illustrates that the composition inhibits insulin receptor signaling activity in the cell line as shown by a colorimetric ELISA assay for tyrosine kinase (K-LISA PTK Screening Kit, Calbiochem cat#539701).

Referring to Figure 1, wells 1 and 2 (in duplicate) contain cell lysate alone, wells 3 and 4 (in duplicate) contain cell lystate contacted with the composition, wells 5 and 6 (in duplicate) contain cell lystate and insulin, and wells 7 and 8 (in duplicate) contain cell lystate and insulin in contact with the composition. As shown by dark and light staining pattern of the wells, wells 1 and 2 had basal tyrosine kinase activity for the 3T3-L1 cell lysate, whereas the addition of the composition to wells 3 and 4 resulted in inhibition of basal tyrosine kinase activity in wells 3 and 4. When insulin was added to the adipocyte cell lysate (see wells 5 & 6), insulin receptor tyrosine kinase activity was much stronger. Addition of the composition (see wells 7 and 8) abolished most of the insulin induced insulin receptor tyrosine kinase activity.

### Human Trial #1

A composition was prepared by the following method: Cow milk was incubated with EDTA (at final EDTA concentration of 10mM) for 1 hour. EDTA was removed by dialysis against de-ionized water for 3 hours. The EDTA-treated milk was incubated with zinc acetate (at final Zn concentration of 50 mM) for 18 hours. The precipitates were removed by centrifugation. Excess zinc was removed by three rounds of dialysis against de-ionized water, 4 hrs, 4 hrs, and 18 hrs respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa. Proteins were precipitated, dried, and packaged into gelatin capsules.

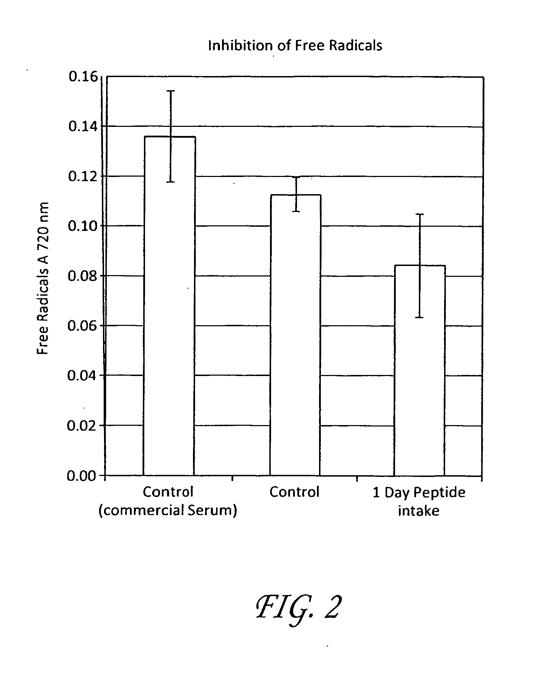

To assess the therapeutic effect on human serum antioxidant levels one day after ingesting the composition, the following experiment was performed: Before the experiment started, blood was collected from the human subject as control serum. Another control serum from commercial source was also used as a reference. The human subject then ingested 200 mg of the composition in the capsules at 7:00 a.m. Fasting blood sample was collected at 9:00 a.m. the following morning. Serum was isolated using centrifugation. The control and experimental serum were tested for the ability to inhibit free radical formation in vitro using an Antioxidant Assay kit from Cayman Chemical according to manufacturer instruction. The level of free radical inhibition was measured in a colorimetric assay at A720nm and the results are shown in Figure 2, shown as mean +/- standard deviation with N=3. As Figure 2 illustrates, the human subject exhibited greater free radical inhibition approximately 26 hours after ingestion of the composition relative to both the commercial serum, and the human subject's own serum prior to ingestion of the composition.

### Human Trial #2

A composition was prepared by the following method: Cow milk was incubated with EDTA (at final EDTA concentration of 10mM) for 1 hour. Excess EDTA was removed by dialysis against de-ionized water for 3 hours. The EDTA -treated milk was incubated with zinc acetate (at final Zn concentration of 50 mM) for 18 hours. The precipitates were removed by centrifugation. Excess zinc was removed by three rounds of dialysis against de-ionized water, 4hrs, 4hrs, and 18 hrs and respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa. Proteins were precipitated, dried, and packaged into gelatin capsules.

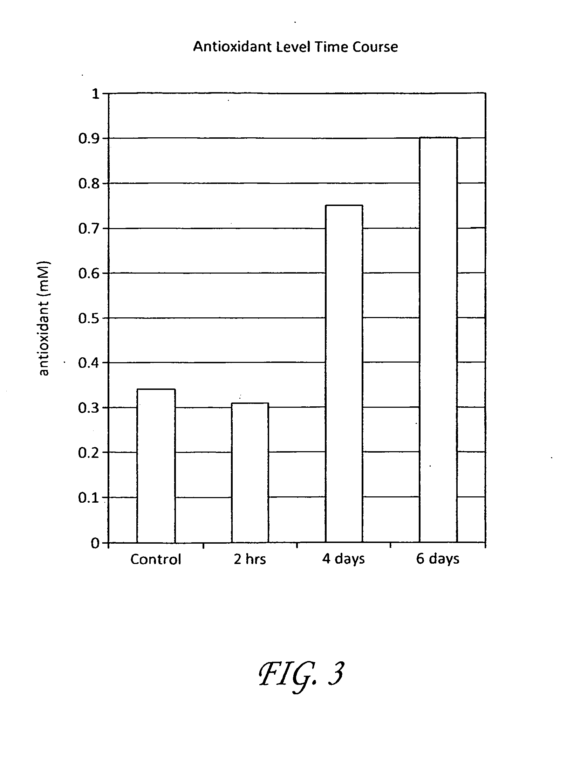

To assess the therapeutic effect on human serum antioxidant levels four days and six days after ingesting the composition, the following experiment was performed: Blood was collected from the human subject as control serum. For six consecutive days, the human subject ingested 200 mg of the composition in a capsule at 7:00 a.m. every morning. Fasting blood was collected at 9:00 a.m. at Day 1 (2 hours after first ingestion), Day 4, and Day 6. Serum was isolated using centrifugation and total serum antioxidant level was measured in duplicates using the Antioxidant Assay kit from Cayman Chemical according to manufacturer instruction. The measured serum antioxidant levels are shown in Figure 3. Although serum antioxidant levels decrease slightly 2 hours after ingestion on Day 1, on Day 4 serum antioxidant levels have roughly doubled relative to the control, and on Day 6 serum antioxidant levels have roughly tripled.

### Human Trial #3

A composition was prepared by the following method: Cow milk was incubated with EDTA (at final EDTA concentration of 10mM) for 1 hour. Excess EDTA was removed by dialysis against de-ionized water for 3 hours. The EDTA -treated milk was incubated with zinc acetate (at final Zn concentration of 50 mM) for 18 hours. The precipitates were removed by centrifugation. Excess zinc was removed by two rounds of dialysis against de-ionized water, 4hrs, 4hrs, and 18 hrs respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa. Proteins were precipitated, dried, and packaged into gelatin capsules.

To assess whether the composition exhibits increased antioxidant activity through increased activity of superoxide dismutase, the following experiment was performed: Prior to the experimental phase, fasting blood was collected at 9:00 a.m. from the human subject as a control sample. For six consecutive days, the human subject ingested 250 mg of the composition in a capsule at 7:00 a.m. every morning. Fasting blood was collected at 9:00 a.m. at Day 6. Blood cells were isolated by removal of serum after centrifugation. Intracellular Cu/Zn superoxide dismutase (SOD) activity was measured by using Superoxide Dismutase Activity Assay from Northwest Life Science according to manufacturer instructions. Blood cell lysate was diluted 25-fold and 500-fold respectively, and SOD inhibition of auto-oxidation reaction was measured in a time course.

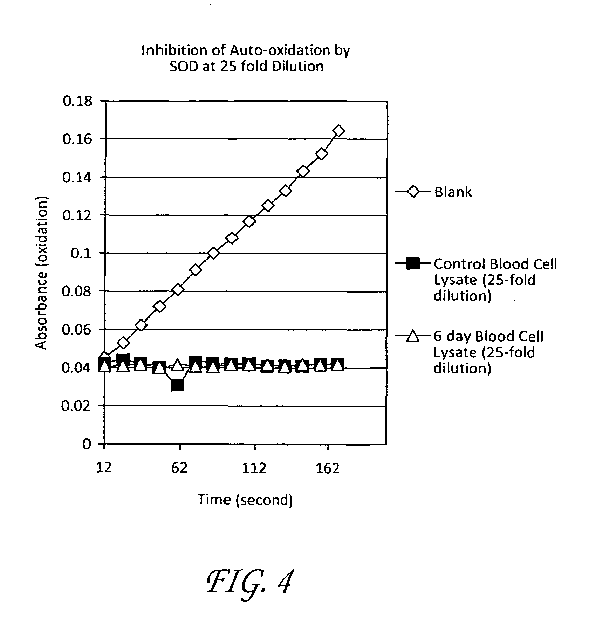

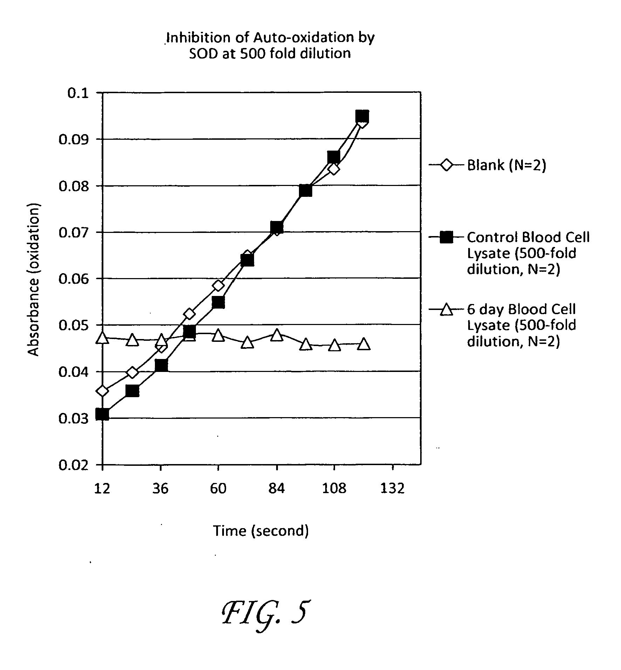

The measured effect on superoxide dismutase activity for the 25-fold dilution sample and the 500-fold dilution sample are shown in Figures 4 and 5, respectively. As Figure 4 shows, at 25-fold dilution, both the control blood cell lysate and the 6-day blood cell lysate (i.e. the lysate obtained after ingestion of the composition for 6 days) strongly inhibit auto-oxidation. As Figure 5 shows, at 500-fold dilution, no SOD activity was detected in the presence of the control blood cell lysate, whereas, in the presence of the 6-day blood cell lysate, significant SOD activity was detected-i.e. significant inhibition of auto-oxidation over time.

### Human Trial #4: Midterm Effects

A composition was prepared by the following method: Cow milk was incubated EDTA (at final EDTA concentration of 10mM) for 1 hour. EDTA was removed by dialysis against de-ionized water for 3 hours. The EDTA-treated milk was incubated with zinc acetate (at final Zn concentration of 50 mM) for 18 hours. The precipitates were removed by centrifugation. Excess zinc was removed by three rounds of dialysis against de-ionized water, 4hrs, 4hrs, and 18 hrs respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa.

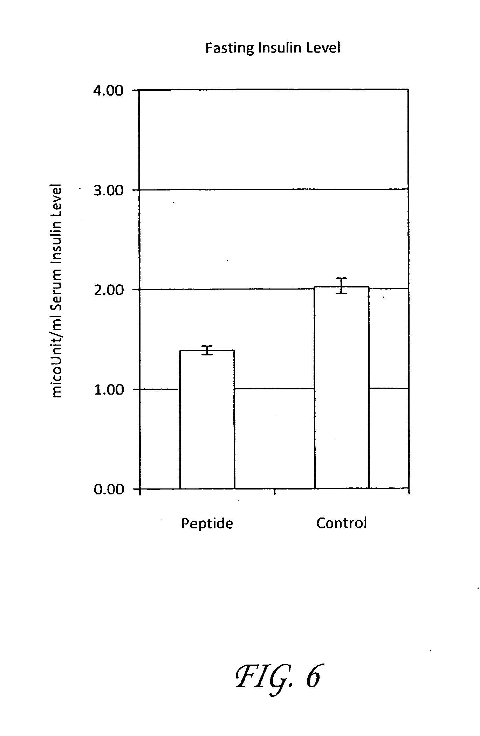

To assess the therapeutic effect on fasting human serum insulin levels the following experiment was performed: during the experimental phase the human subject ingested 20 ml (10mg/ml) liquid formulation, of the composition for 25 days. During the control phase, the human subject was monitored while ingesting no composition of the invention for one month. Blood samples were taken at 3 different time points during the month. Each sample (in duplicate) was tested for fasting insulin and fed insulin level using ELISA assays. As can be seen from Figure 6, ingestion of the composition significantly lowered the subject's fasting insulin levels. The fasting insulin level during the experimental phase was 1.38 +/- 0.04 µU/mL The insulin level the control phase was 2.02 +/- 0.07 µU/ml. The composition of the invention lowered the subject's insulin level by about 32%.

In addition, though not shown in the figure, the fed insulin level during the experimental phase was 30 +/- 13.65 µU/ml. The fed insulin level of the control phase was 22 +/- 7.21 µU/ml. Thus, the composition of the invention did not significantly alter fed insulin level. This suggests that the composition does not indiscriminately inhibit release of insulin. In the absence of food, the composition lowers circulating insulin in the blood; whereas insulin is released quickly after a meal. This phenomenon implies that the compositions of the invention increases a subject's insulin sensibility.

### Human Trial #5:

A composition was prepared by the following method: Cow milk was incubated EDTA (at final EDTA concentration of 10mM) for 1 hour. EDTA was removed by dialysis against de-ionized water for 3 hours. The EDTA -treated milk was incubated with zinc acetate (at final Zn concentration of 50 mM) for 18 hours. The precipitates were removed by centrifugation. Excess zinc was removed by three rounds of dialysis against de-ionized water, 4hrs, 4hrs, and 18 hrs respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa.

To assess the therapeutic effect on serum antioxidant levels and serum free radical levels over a period of approximately one month, the following experiment was performed: During the experimental phase, the human subject ingested 20ml (10mg/ml), liquid form, of the composition for 25 days. During the control phase, the human subject was monitored while ingesting no quantity of the composition for one month. Blood samples were taken at 3 different time points during the month. Each sample (in duplicate) was tested for anti-oxidant level using a Calbiochem Total Antioxidant Status Assay Kit, Urine samples were also taken at 3 different time points during each of the two phases to determine levels of free radicals using an OXIDATA Oxidation Stress Test.

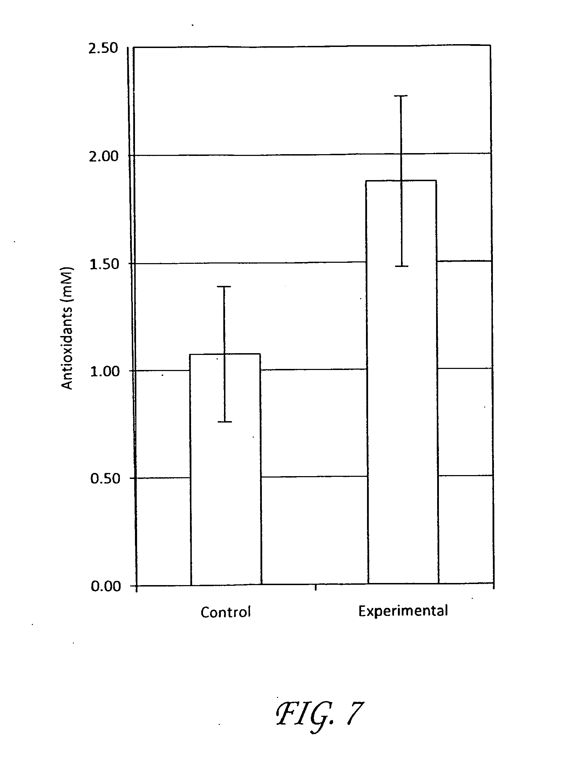

As shown in Figure 7, the antioxidant level during the control phase was 1.07 +/- 0.32 mM. The antioxidant level during the experimental phase was 1.88 +/-0.39 mM. The composition of the invention increased the subject's antioxidant level by approximately 76%.

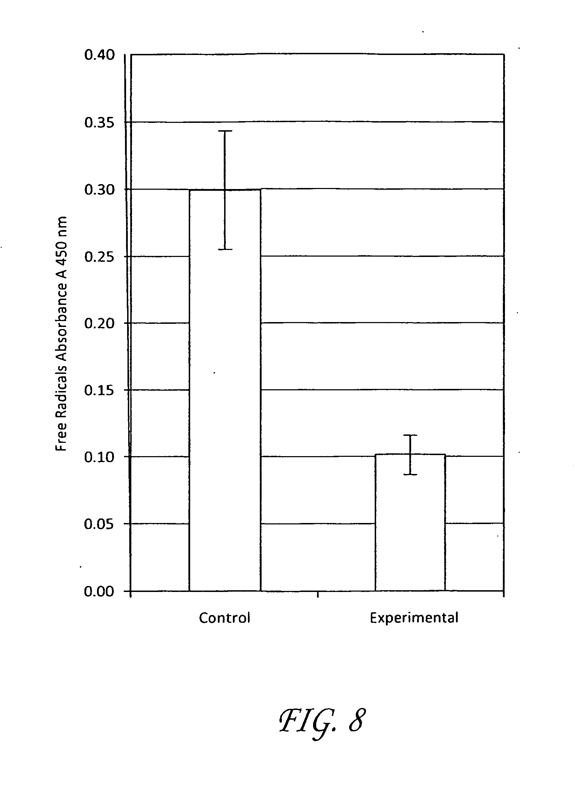

As shown in Figure 8, the free radical level during the control phase was measured by a colorimetric assay. The absorbance at A450 was 0.30 +/- 0.04. The free radicals level during the experimental phase was 0.10 +/- 0.02. The composition of the invention lowered the subject's free radicals in the urine by approximately 66%.

### Human Trial #6: Long Term Effects

Over a period of three years, a human subject repeatedly ingested several compositions comprising zinc-charged peptide fragments derived from mammalian serum and milk. The compositions were administered in various formulations. For example, the human subject ingested: bovine and porcine serum peptide in liquid form or capsule form, ranging from 5 mg - 250 mg per day; cow milk peptides in liquid form, capsule form, or dry powder without capsule, ranging from 10mg - 450mg per day; and zinc-fetuin peptide fragments in liquid or capsule form, ranging from 1mg - 10mg per day. The various compositions and formulations were ingested on approximately 75% or more of the days making up the three year period. Despite the variation in frequency of ingestion, a composition was always ingested by the human subject at least once in a three week period. In other words, the longest gap in time between subsequent ingestions was less than 3 weeks.

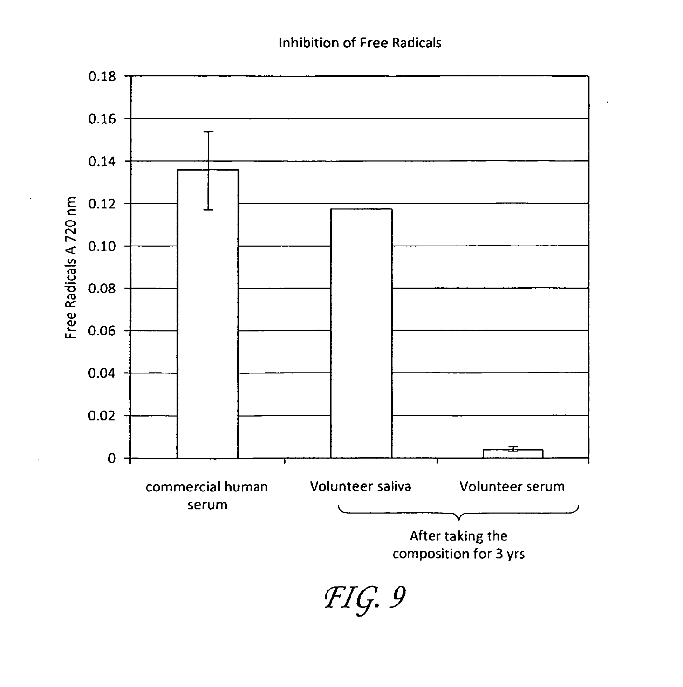

Three years from the first date of taking the peptide, the human subject fasted overnight. Saliva and blood serum were collected at 7:00 a.m. in the morning. The control and experimental samples were tested for the ability to inhibit free radical formation in vitro by using an Antioxidant Assay kit from Cayman Chemical according to manufacturer instruction. Figure 9 displays the level of free radical inhibition as measured by colorimetric assay at A720nm, N=3. In comparison to a normal person, the volunteer's saliva has a free radical level similar to the level in a normal person's serum. In contrast, the subject's serum free radical level is much lower, indicating that the subject's serum possesses a strong antioxidant power sufficient to neutralize a high percentage of the free radical species.

### C. Elegans Longevity Study

To determine the effect of the composition on the lifespan of C. *elegans,* a composition was prepared by the following method: Cow milk was incubated with EDTA (at final EDTA concentration of 20mM) for 1 hour. EDTA was removed by dialysis against de-ionized water for 3 hours. The EDTA -treated milk was incubated with zinc acetate (at final Zn concentration of 50 mM) for 18 hours. The precipitates were removed by centrifugation. Excess zinc was removed by two rounds of dialysis against de-ionized water, 18 hrs and 4 hrs respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa.

The composition was then the subject of a *C*. *elegans* longevity study conducted by the University of Utah. In the study, a control group of40 L4 larval N2 (wild type) worms were cultured on 4 standard NGM agar plates and an experimental group of 40 L4 larval N2 (wild type) worms were cultured on 4 NGM plates containing 1:500 dilution of 10mg/ml peptide in liquid form. All plates were seeded with *E*. *coli* strain OP50 and stored at 4 °C until needed. Worms were transferred to fresh plates every day until progeny production ceased, after which transfers were performed weekly.

The mean number of surviving animals for each day was recorded. Worms were assessed as being dead based on the lack of response to prodding and the absence of pharyngeal pumping. Using the mean values, the fraction of surviving animals was calculated. In addition, the number of animals that died on each day was calculated and the values were used in log rank tests to examine statistically significant differences between the values obtained for N2 worms grown on standard plates and N2 worms cultured on composition of invention.

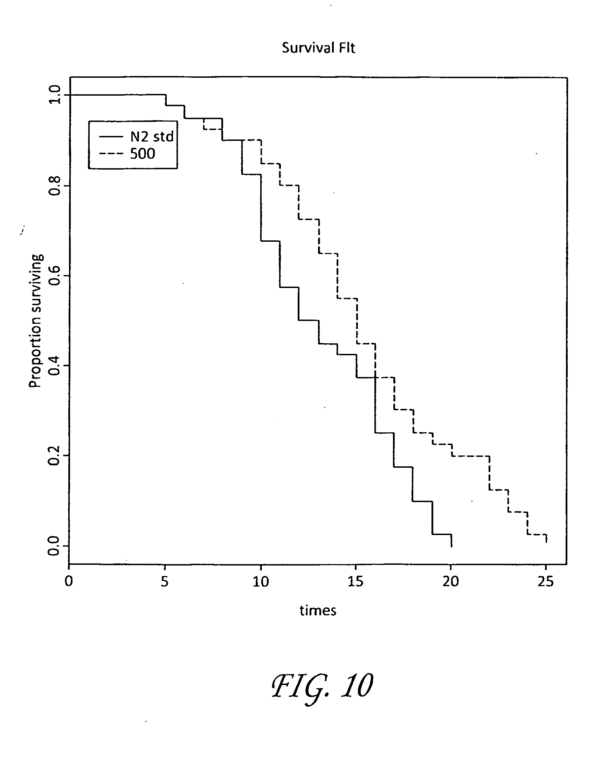

The study showed that ingestion of the composition by *C*. *elegans* showed a weak but statistically significant increase in life span. As illustratedin Figure 10, in the control group, 50% of worms died at Day 14, whereas 50% worms that ingested the compositino died at Day 17. This is an increase in median lifespan by 21 %. The last control worm died at Day 20, compare to the last worm that ingested the peptides died at Day 25. Assuming the average human lifespan is 75 years, this composition may potentially increase the lifespan by 15 years.

One possible explanation relates to the *daf-2* gene which encodes an insulin-like receptor in C. *elegans.* Mutations in *daf-2* have been shown by researchers to double the lifespan of the worms. The gene is known to regulate reproductive development, aging, resistance to oxidative stress, thermotolerance, resistance to hypoxia, and also resistance to bacterial pathogens. Therefore, it may be the case that the zinc-charged peptide fragments modify expression of the *daf-2* gene which acts to regulate aging in C. *elegans.*

### Human Trial #7: Effects on Serum Ghrelin Level

A composition was prepared by the following method: Cow milk was incubated EDTA (at final EDTA concentration of 10mM) for 1 hour. Excess EDTA was removed by dialysis against de-ionized water for 3 hours. The EDTA -treated milk was incubated with zinc acetate (at final Zn concentration of 50 mM) for 18 hours. The precipitates were removed by centrifugation. Excess zinc was removed by three rounds of dialysis against de-ionized water, 4hrs, 4hrs, and 18 hrs respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa. Proteins were precipitated, dried, and packaged into gelatin capsules.

To determine the effects of the composition on serum ghrelin levels upon administering to a human subject, the following experiment was performed: Prior to the experimental phase, blood was collected from the human subject to be used as a control serum. For six consecutive days, the human subject ingested 200 mg of the composition in capsule form at 7:00 a.m. every morning. On Day 4 and Day 6, fasting blood was collected at 9:00 a.m. Serum was isolated using centrifugation. Each sample (in triplicate) was tested for ghrelin level using a LINCO Active Ghrelin ELISA Kit.

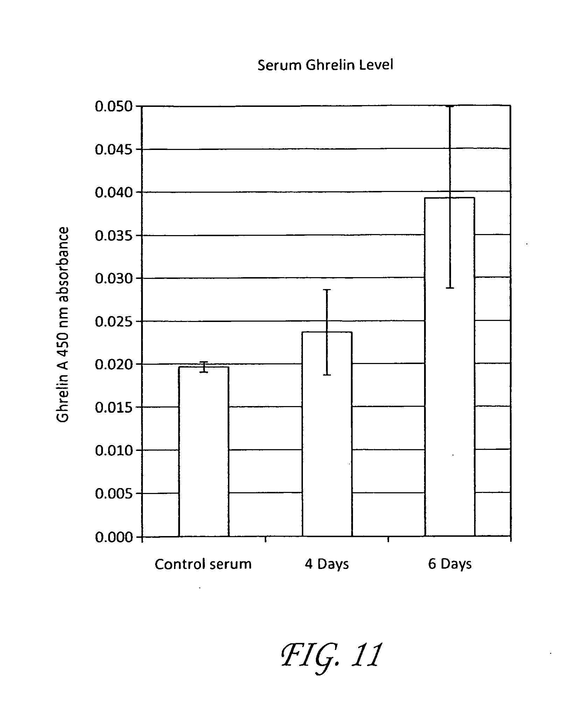

As shown in Figure 11, the serum ghrelin level of the subject prior to taking the composition (i.e, the control) had an A450nm absorbance reading of 0.019+/- 0.001. The serum ghrelin level during the experimental phase was 0.024 +/- 0.005 and 0.039 +/- 0.01. Thus, the composition statistically significantly increased the subject's serum ghrelin level, and furthermore, has an increasing trend over the six day period.

### Human Trial #8: Effects on Serum TNF-Alpha Level

A composition was prepared by the following method: Cow milk was incubated with EDTA (at final EDTA concentration of 10mM) for 1 hour. The EDTA was removed by dialysis against de-ionized water for 3 hours. The EDTA -treated milk was incubated with zinc acetate (at final Zn concentration of 50 mM) for 18 hours. The precipitates were removed by centrifugation. Excess zinc was removed by three rounds of dialysis against do-ionized water, 4hrs, 4hrs, and18 hrs respectively. Zinc-charged milk prepared as described above was incubated with papain (60 unit per milliliter milk mixture, from papaya latex) for 2 hours at 37°C. The fragments were collected by passing the incubation mixture through a molecular sieve with a molecular weight cut-off at 10 KDa. Proteins were precipitated, dried, and packaged into gelatin capsules.

To determine the effects of the composition on serum TNF-alpha levels upon administering to a human subject, the following experiment was performed: Prior to the experimental phase, blood was collected from the human subject as control serum. For six consecutive days, the human subject ingested 200 mg of the composition in capsule form at 7:00 a.m. every morning. On Day 4 and Day 6, fasting blood was collected at 9:00 a.m. Serum was isolated using centrifugation. Serum TNF-alpha level was determined using an Endogen Human TNF-alpha ELISA kit from Pierce Biotechnology, Inc., according to manufacturer instruction.

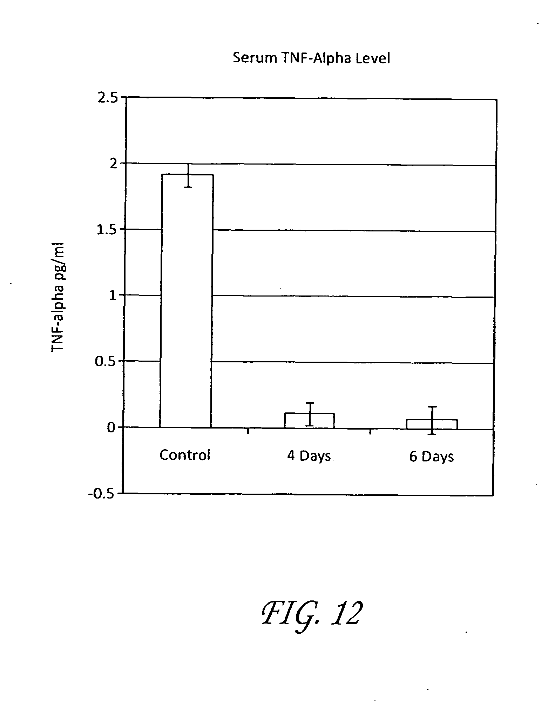

As shown in Figure 12, the serum TNF-alpha level of the subject prior to taking the composition (i.e. the control) is in the normal range of 1.8 pg/ml. After ingesting the composition daily for 4 days and 6 days, the subject's serum TNF-alpha levels decreased to 0.15 pg/ml and 0.18 pg/ml, respectively. This result is in alignment with the increase of serum ghrelin levels induced by the composition of invention, since it has been shown that ghrelin can inhibit secretion of pro-inflammatory factors, including TNF-alpha (Am J Physiol Endocrinol Metab. 293:1697-702,2007).

### Human Trial #9: Effects on Serum Triglycerides Level

A composition was prepared by the following method: Cow milk was incubated with EDTA (at final EDTA concentration of 10mM) for 1 hour. Excess EDTA was removed by dialysis against de-ionized water for 3 hours. The EDTA-treated milk was incubated with zinc acetate (at final Zn concentration of 50 mM) for 18 hours. The precipitates were removed by centrifugation. Excess zinc was removed by three rounds of dialysis against de-ionized water, 4hrs, 4hrs, and 18 hrs respectively. Zinc-charged milk, prepared as described above, was incubated with papain (60 unit per milliliter milk mixture, from papaya lastex) for 2 hours at 37°C. The fragments were collected by passing the incubation mixture through a molecular sieve with a molecular weight cut-off at 10 KDa. Proteins were precipitated, dried, and packaged into gelatin capsules.

To determine the effects of the composition on serum triglycerides levels upon administering to a human subject, the following experiment was performed: Prior to the experimental phase, blood was collected from the human subject to be used as a control serum. For eight consecutive days, the human subject ingested 200 mg of the composition in capsule form at 7:00 a.m. every morning. On Day 9, fasting blood was collected at 9:00 a.m. Serum was isolated using centrifugation. On Day 11 (two days without ingestion of composition), fasting blood was collected at 9:00 a.m. Serum was isolated using centrifugation. On Day 19 (10 days without ingestion of composition), fasting blood was collected at 9:00 a.m. Serum was isolated using centrifugation. Each sample (in triplicate) was tested for triglycerides level using Cardiochek™ triglycerides testing strips and Analyzer.

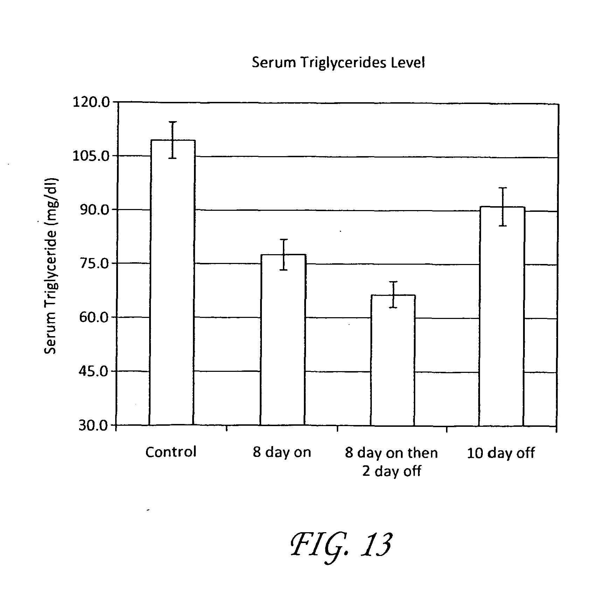

As shown in Figure 13, the serum triglycerides level of the subject prior to taking the composition (i.e. the control) was 109 +/- 5.1 mg/dl. The serum triglycerides level during the experimental phase was 77.3 +/- 4.2 mg/dl. Serum triglycerides levels two days and 10 days without ingestion of composition were 66.3 +/-3.5 mg/dl, and 91.0 +/- 5.3 mg/dl. Thus, the composition statistically significantly decreased the subject's serum triglycerides level. The triglycerides lowering effect continued for at least two days without further daily ingestion of the composition, and the triglycerides level returned to higher level after 10 day wash out period.

## Claims

1. A composition for use for treating oxidative stress of cancer patients undergoing chemotherapy by increasing serum antioxidant concentrations in a mammalian subject comprising a first plurality of zinc-charged protein fragments of a first zinc-binding protein.

2. The composition of Claim 1, wherein the first plurality of zinc-charged protein fragments are obtainable by a process comprising the following steps in either order:
fragmenting the first zinc-binding protein; and
contacting the zinc-binding protein or a fragment thereof with zinc ion.

3. The composition of Claims 1 or 2, wherein the composition is a dietary supplement, a food additive or food composition, a pharmaceutical composition or a nutraceutical composition.

4. The composition of any of Claims 1 through 3, wherein the composition additionally comprises a second plurality of zinc-charged protein fragments created by a process comprising fragmenting a second zinc-binding protein.

5. The composition of any of Claim 1 through 4, wherein the first zinc-binding protein and the second zinc-binding protein, if present, are selected from a group consisting of α -2-HS-glycoprotein, α-1-acid glycoprotein, α-1-antitrypsin, albumin, transferrin and α- fetoprotein.

6. The composition of Claim 4, wherein the composition additionally comprises a third plurality of zinc-charged protein fragments created by a process comprising fragmenting a third zinc-binding protein, and wherein each of the first zinc-binding protein, second zinc-binding protein, and third zinc-binding protein are selected from a group consisting of α -2-HS-glycoprotein, α-1-acid glycoprotein, α-1-antitrypsin, albumin, transferrin and α- fetoprotein.

7. The composition of any of Claims 1 through 6 in unit dosage form, wherein the mammal is a human and the unit dosage is 1.4 mg.

8. The composition of any of Claims 1 through 6 in a daily dosage form, wherein the mammal is a human and the daily dosage is 3.5 g.

9. A composition according to Claim 1, the composition obtainable by a preparation process comprising the following steps in any order:
contacting a zinc-binding serum or milk protein or fragment thereof with a chelating agent;
contacting the protein or fragment thereof with zinc ion; and
fragmenting the protein or further fragmenting the fragments thereof.

10. The composition of Claim 9, wherein the preparation process further comprises removing the chelating agent.

11. The composition of Claim 9 or 10, wherein the preparation process further comprises removing excess zinc not bound to any protein.

12. A composition for use for treating cachexia by increasing serum ghrelin concentrations in a mammalian subject comprising a first plurality of zinc-charged protein fragments of a first zinc-binding protein, the composition obtainable by a process comprising the following steps in either order:
fragmenting the first zinc-binding protein; and
contacting the zinc-binding protein or a fragment thereof with zinc ion.

13. A composition for use for treating TNF-alpha related inflammation by decreasing serum TNF-alpha concentrations in a mammalian subject comprising a first plurality of zinc-charged protein fragments of a first zinc-binding protein, the composition obtainable by a process comprising the following steps in either order:
fragmenting the first zinc-binding protein; and
contacting the zinc-binding protein or a fragment thereof with zinc ion.

## Patentansprüche

1. Zusammensetzung zur Verwendung bei der Behandlung von oxidativem Stress bei Krebspatienten unter Chemotherapie durch Erhöhen der Antioxidationsmittelkonzentrationen in Serum bei einem Säugetier-Individuum, die eine erste Mehrzahl zinkbeladener Proteinfragmente eines ersten zinkbindenden Proteins umfasst.

2. Zusammensetzung nach Anspruch 1, wobei die erste Mehrzahl von zinkbeladenen Proteinfragmenten durch ein Verfahren erhältlich ist, das die folgenden Schritte in beliebiger Reihenfolge umfasst:
Fragmentieren des ersten zinkbindenden Proteins; und
Inkontaktbringen des zinkbindenden Proteins oder eines Fragments davon mit Zinkion.

3. Zusammensetzung nach Anspruch 1 oder 2, wobei die Zusammensetzung ein Nahrungsergänzungsmittel, ein Lebensmittelzusatzstoff oder eine Lebensmittelzusammensetzung, eine pharmazeutische Zusammensetzung oder eine nutrazeutische Zusammensetzung ist.

4. Zusammensetzung nach einem der Ansprüche 1 bis 3, wobei die Zusammensetzung darüber hinaus eine zweite Mehrzahl von zinkbeladenen Proteinfragmenten umfasst, die durch ein Verfahren hergestellt wurden, das das Fragmentieren eines zweiten zinkbindenden Proteins umfasst.

5. Zusammensetzung nach einem der Ansprüche 1 bis 4, wobei das erste zinkbindende Protein und das zweite zinkbindende Protein, falls vorhanden, aus einer Gruppe ausgewählt sind, bestehend aus einem α-2-HS-Glykoprotein, einem α-1-Säure-Glykoprotein, einem α-1-Antitrypsin, Albumin, Transferrin und α-Fetoprotein.

6. Zusammensetzung nach Anspruch 4, wobei die Zusammensetzung darüber hinaus eine dritte Mehrzahl von zinkbeladenen Proteinfragmenten umfasst, die durch ein Verfahren hergestellt wurden, das das Fragmentieren eines dritten zinkbindenden Proteins umfasst, und wobei jedes des ersten zinkbindenden Proteins, des zweiten zinkbindenden Proteins und des dritten zinkbindenden Proteins aus einer Gruppe ausgewählt ist, bestehend aus einem α-2-HS-Glykoprotein, einem α-1-Säure-Glykoprotein, einem α-1-Antitrypsin, Albumin, Transferrin und α-Fetoprotein.

7. Zusammensetzung nach einem der Ansprüche 1 bis 6 in Einheitsdosis-Darreichungsform, wobei das Säugetier ein Mensch ist und die Einheitsdosis 1,4 mg beträgt.

8. Zusammensetzung nach einem der Ansprüche 1 bis 6 in Tagesdosis-Darreichungsform, wobei das Säugetier ein Mensch ist und die Tagesdosis 3,5 g beträgt.

9. Zusammensetzung nach Anspruch 1, wobei die Zusammensetzung mit einem Herstellungsverfahren erhältlich ist, das die folgenden Schritte in einer beliebigen Reihenfolge umfasst:
Inkontaktbringen eines zinkbindenden Serums oder Milchproteins oder Fragments davon mit einem Chelatbildner;
Inkontaktbringen des Proteins oder Fragments davon mit Zinkion; und
Fragmentieren des Proteins oder weiteren Fragmentieren der Fragmente davon.

10. Zusammensetzung nach Anspruch 9, wobei das Herstellungsverfahren darüber hinaus das Entfernen des Chelatbildners umfasst.

11. Zusammensetzung nach Anspruch 9 oder 10, wobei das Herstellungsverfahren darüber hinaus das Entfernen von überschüssigem Zink umfasst, das nicht an Protein gebunden ist.

12. Zusammensetzung zur Verwendung bei der Behandlung von Kachexie durch Erhöhen der Ghrelinkonzentrationen in Serum bei einem Säugetier-Individuum, die eine erste Mehrzahl von zinkbeladenen Proteinfragmenten eines ersten zinkbindenden Proteins umfasst, wobei die Zusammensetzung mit einem Verfahren erhältlich ist, das die folgenden Schritte in einer beliebigen Reihenfolge umfasst:
Fragmentieren des ersten zinkbindenden Proteins; und
Inkontaktbringen des zinkbindenden Proteins oder eines Fragments davon mit Zinkion.

13. Zusammensetzung zur Verwendung bei der Behandlung einer mit TNF-alpha in Zusammenhang stehenden Entzündung durch Senken der TNF-alpha-Konzentrationen in Serum bei einem Säugetier-Individuum, die eine erste Mehrzahl von zinkbeladenen Proteinfragmenten eines ersten zinkbindenden Proteins umfasst, wobei die Zusammensetzung mit einem Verfahren erhältlich ist, das die folgenden Schritte in einer beliebigen Reihenfolge umfasst:
Fragmentieren des ersten zinkbindenden Proteins; und
Inkontaktbringen des zinkbindenden Proteins oder eines Fragments davon mit Zinkion.

## Revendications

1. Composition destinée à être utilisée pour traiter le stress oxydatif chez des patients cancéreux subissant une chimiothérapie en augmentant les concentrations d'antioxydant dans le sérum chez un sujet mammifère, comprenant une première pluralité de fragments protéiques chargés en zinc d'une première protéine se liant au zinc.

2. Composition selon la revendication 1, dans laquelle la première pluralité de fragments protéiques chargés en zinc peut être obtenue par un processus comprenant les étapes suivantes dans un ordre quelconque :
la fragmentation de la première protéine se liant au zinc ; et
la mise en contact de la protéine se liant au zinc ou d'un fragment de celle-ci avec un ion zinc.

3. Composition selon les revendications 1 ou 2, où la composition est un complément diététique, un additif alimentaire ou une composition alimentaire, une composition pharmaceutique ou une composition nutraceutique.

4. Composition selon l'une quelconque des revendications 1 à 3, où la composition comprend en outre une deuxième pluralité de fragments protéiques chargés en zinc créée par un processus comprenant la fragmentation d'une deuxième protéine se liant au zinc.

5. Composition selon l'une quelconque des revendications 1 à 4, dans laquelle la première protéine se liant au zinc et la deuxième protéine se liant au zinc, si elle est présente, sont sélectionnées dans un groupe consistant en l'α-2-HS-glycoprotéine, l'α-1-glycoprotéine acide, l'α-1-antitrypsine, l'albumine, la transferrine et l'α-foetoprotéine.

6. Composition selon la revendication 4, où la composition comprend en outre une troisième pluralité de fragments protéiques chargés en zinc créée par un processus comprenant la fragmentation d'une troisième protéine se liant au zinc, et où chacune de la première protéine se liant au zinc, la deuxième protéine se liant au zinc, et la troisième protéine se liant au zinc sont sélectionnées dans un groupe consistant en l'α-2-HS-glycoprotéine, l'α-1-glycoprotéine acide, l'α-1-antitrypsine, l'albumine, la transferrine et l'α-foetoprotéine.

7. Composition selon l'une quelconque des revendications 1 à 6 sous forme de dose unitaire, où le mammifère est un humain et la dose unitaire est de 1,4 mg.

8. Composition selon l'une quelconque des revendications 1 à 6 sous forme de dose quotidienne, où le mammifère est un humain et la dose quotidienne est de 3,5 g.

9. Composition selon la revendication 1, la composition pouvant être obtenue par un processus de préparation comprenant les étapes suivantes dans un ordre quelconque :
la mise en contact d'une protéine sérique ou de lait se liant au zinc ou d'un fragment de celle-ci avec un agent de chélation ;
la mise en contact de la protéine ou d'un fragment de celle-ci avec un ion zinc ; et
la fragmentation de la protéine ou la fragmentation supplémentaire des fragments de celle-ci.

10. Composition selon la revendication 9, où le processus de préparation comprend en outre l'élimination de l'agent de chélation.

11. Composition selon la revendication 9 ou 10, où le processus de préparation comprend en outre l'élimination du zinc en excès non lié à une quelconque protéine.

12. Composition destinée à être utilisée pour traiter la cachexie en augmentant les concentrations de ghréline dans le sérum chez un sujet mammifère, comprenant une première pluralité de fragments protéiques chargés en zinc d'une première protéine se liant au zinc, la composition pouvant être obtenue par un processus comprenant les étapes suivantes dans un ordre quelconque :
la fragmentation de la première protéine se liant au zinc ; et
la mise en contact de la protéine se liant au zinc ou d'un fragment de celle-ci avec un ion zinc.

13. Composition destinée à être utilisée pour traiter une inflammation associée au TNF-alpha en réduisant les concentrations de TNF-alpha dans le sérum chez un sujet mammifère, comprenant une première pluralité de fragments protéiques chargés en zinc d'une première protéine se liant au zinc, la composition pouvant être obtenue par un processus comprenant les étapes suivantes dans un ordre quelconque :
la fragmentation de la première protéine se liant au zinc ; et
la mise en contact de la protéine se liant au zinc ou d'un fragment de celle-ci avec un ion zinc.
